# EUROPEAN PATENT APPLICATION

(11) **EP 4 585 258 A1**
(43) Date of publication of application: **16.07.2025**
(21) Application number: 23863284.8
(22) Date of filing: 11.09.2023
(51) Int. Cl.: A61N 5/06

(54) **LIGHT RADIATION SYSTEM, LIGHT RADIATION METHOD, LIGHT MEASUREMENT METHOD, AND LIGHT MEASUREMENT DEVICE**

(30) Priority: 09.09.2022 JP 2022143754
(71) Applicant: Tsubota Laboratory, Inc., Tokyo 160-8582 (JP)
(72) Inventor: TSUBOTA, Kazuo, Tokyo 160-8582 (JP); KONDO, Shinichiro, Tokyo 160-8582 (JP)
(74) Representative: Mewburn Ellis LLP
(86) International application number: PCT/JP2023/033110
(87) International publication number: WO 2024/053746

(57) **Abstract**

To provide a light radiation system, a light radiation method, a light measurement method, and a light measurement device advantageous to activation of OPN5 present in a retina of a person.

The above-described problem is solved by a light radiation system comprising one or two or more light sources that radiate light having a wavelength in a wavelength region overlapping a peak wavelength of an OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated. Further, the above-described problem can also be solved by a light radiation system comprising a light radiating means including the one or two or more light sources and a light measuring means for measuring spectral data of the light and comparing the spectral data of the light and data of the OPN5 absorption spectrum, and configured to regulate an installation position and the like of each light source on the basis of results obtained by the comparison.

## Description

### Field of the Invention

The present invention relates to a light radiation system, a light radiation method, a light measurement method, and a light measurement device.

### BACKGROUND ART

In recent years, the effects of light on a human body have been examined from various perspectives, and reports based on new findings have been made. For example, it has been reported that circadian rhythm is improved by exposure to sunlight (Non-Patent Document 1), light emitted from light-emitting diode (LED) lighting, a liquid crystal display that uses an LED as a backlight, or the like has a significant effect on the body and mind (Non-Patent Document 2), violet light prevents myopia and suppresses an onset of myopia (Patent Document 1), and the like. In particular, the present inventors have recently reported on the effects of violet light on myopia. For example, in Patent Document 1 and Non-Patent Document 3, it is proposed that light having a specific wavelength is effective in preventing and suppressing the progression of myopia, and there are great expectations in recent years with the number of persons with myopia still increasing worldwide.

Opsin 5 (OPN5) is known as a photoreceptor protein, and research on visual functions and non-visual functions in humans and animals is underway (Non-Patent Document 4).

### PRIOR ART DOCUMENTS

### Non-Patent Documents

Non-Patent Document 1: Megumi Hatori, Kazuo Tsubota, Anti-Aging Medicine - Journal of Japanese Society of Anti-aging Medicine, Vol. 11, No. 3, 065 (385) to 072 (392) (2015)
Non-Patent Document 2: Kazuo Tsubota, "Blue Light - Threat to Internal Clock," Shueisha (November 20, 2013)
Non-Patent Document 3: Hidemasa Torii et al., EbioMedicine, "DOI: http://dx.doi.org/10.1016/j.ebiom.2016.12.007"
Non-Patent Document 4: Takahiro Yamashita, "Novel UV Light-sensitive Photoreceptive Protein in Vertebrates," Seibutsu Butsuri 51 (4), 186 to 187 (2011)

### Patent Documents

Patent Document 1: WO2015/186723A1

### SUMMARY OF THE INVENTION

### Problems to be Solved by the Invention

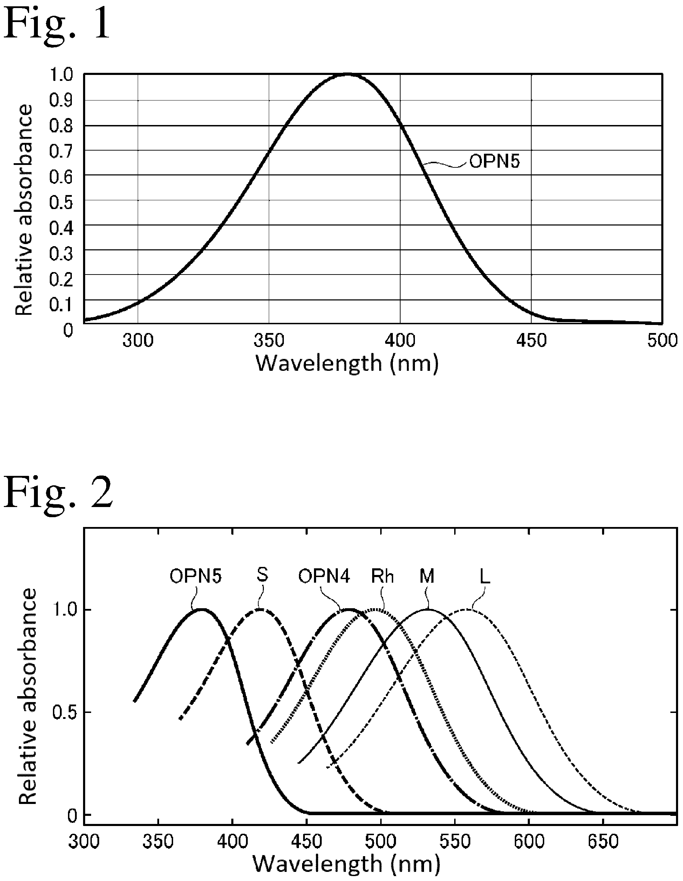

As shown in Fig. 1, OPN5 exhibits an absorption spectrum having a peak at 380 nm. If such OPN5 can be activated, it can be expected that the relationship with visual functions and non-visual functions, as well as with myopia progression suppression, which the present inventors are researching, will be clearly defined.

The present invention has been made to solve the above-described problems. The present invention aims to provide a light radiation system, a light radiation method, a light measurement method, and a light measurement device.

### Means for Solving the Problems

(1) A light radiation system (first light radiation system) according to the present invention comprises one or two or more light sources that radiate light having a wavelength in a wavelength region overlapping a peak wavelength of an OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated.

According to this first light radiation system, each light source is regulated in terms of any one of the above, making it possible to radiate light having a wavelength in a wavelength region overlapping the peak wavelength of the OPN5 absorption spectrum to an irradiated subject. As a result, the first light radiation system is advantageous to activation of OPN5 present in a retina of a person who is the irradiated subject.

(2) A light radiation system (second light radiation system) according to the present invention comprises one or two or more light sources that radiate light having a wavelength in a wavelength region overlapping a peak wavelength of an OPN5 absorption spectrum. A spectrum of the light received by an irradiated subject is regulated so that a received effective spectral irradiance (W/m²/nm) calculated as a product of a spectral irradiance (W/m²/nm) of each wavelength in a wavelength region of 280 nm to 500 nm of the light and a relative absorbance of data of the OPN5 absorption spectrum, on a per wavelength basis, is 0.2% or more of a reference effective spectral irradiance (W/m²/nm) calculated as a product of a spectral irradiance (W/m²/nm) of a sunlight spectrum defined by AM1.5G and a relative absorbance of the OPN5 absorption spectrum, on a per wavelength basis.

According to this second light radiation system, the spectrum of the light received by the irradiated subject is regulated so that the received effective spectral irradiance is 0.2% or more of the reference effective spectral irradiance. As a result, the second light radiation system is advantageous to the activation of OPN5 present in the retina of the person who is the irradiated subject. The light having the irradiance that is a percentage within the above-described range is calculated on the basis of the light spectrum measured at the irradiated subject or a position around the irradiated subject.

(3) A light radiation system (third light radiation system) according to the present invention comprises a light radiating means including one or two or more light sources that radiate light having a wavelength in a wavelength region overlapping a peak wavelength of an OPN5 absorption spectrum, and a light measuring means for measuring spectral data of the light and comparing the spectral data of the light and data of the OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated on the basis of results obtained by the comparison.

According to this third light radiation system, the installation position, the radiation direction of the light, the wavelength range of the light, the irradiance, the radiation time frame, and the radiation duration of each light source are regulated on the basis of the results obtained through comparison by the light measuring means. As a result, the third light radiation system is advantageous to the activation of OPN5 present in the retina of the person who is the irradiated subject.

In the first to third light radiation systems, preferably the light is light including 380 nm that is the peak wavelength of the OPN5 absorption spectrum and, more preferably the light is light having a wavelength of 400 nm or less or 410 nm or less. It should be noted that light having a wavelength of 400 nm or less or 410 nm or less is light having a low relative luminous efficiency, and thus such light is advantageous to facilitating long-period radiation directed toward a person in a case in which the light is constituted solely by wavelengths of 400 nm or less or 410 nm or less or, even if the light includes wavelengths of 400 nm or greater or 410 nm or greater, or the spectral irradiance or the irradiance in the wavelength region is lower than the spectral irradiance or the irradiance in the wavelength region of 400 nm or less or 410 nm or less.

In the first to third light radiation systems, the one or two or more light sources are disposed at one or two or more positions selected from a position where the light is radiated toward an irradiated subject who is a radiation target, a position in a field of view of the irradiated subject, a position along the subject' s visual line, a position where the light is radiated to the irradiated subject as diffused light diffused by a diffusing material, and a position where the light is radiated to the irradiated subject as reflected light reflected by a reflecting material.

In the first to third light radiation systems, for each of the one or two or more light sources, (i) in a case in which the light source is provided at a position where the light is radiated toward the irradiated subject, the irradiance of the light is regulated in accordance with a distance and/or an angle from the light source to the irradiated subject, (ii) in a case in which the light source is provided at a position in the field of view of the irradiated subject or a position in the visual line direction from the irradiated subject side, the irradiance of the light is regulated in accordance with the distance and/or the angle from the light source to the irradiated subject, (iii) in a case in which the light source is provided at a position where the light is radiated to the irradiated subject as the diffused light diffused by the diffusing material, the irradiance of the light is regulated in accordance with a distance and/or an angle from the light source and the diffusing material to the irradiated subject, or a diffusion angle and/or a transmittance of the diffusing material, and (iv) in a case in which the light source is provided at a position where the light is radiated to the irradiated subject as the reflected light reflected by the reflecting material, the irradiance of the light is regulated in accordance with a distance and/or an angle from the light source and the reflecting material to the irradiated subject, or a reflection angle and/or a reflectance of the reflecting material.

In the first to third light radiation systems, each of the one or two or more light sources is selected from a structure-attached light source attached to a floor (including underfoot), a wall, a ceiling (including a suspended ceiling), or other structure; an installation-attached light source attached to a desk, a table, a partition (divider, screen), a shelf, a desktop member, a personal computer (PC), a display, a PC keyboard, a television, an audio device, or other equipment or installation; a built-in or attached light source of a portable PC, a tablet, a smartphone, or other mobile terminal; a body-accessory-attached light source detachably mounted or attached to a vision correcting aid, eye protective equipment, face protective equipment, neck hanging accessory, ear hooking accessory, head-attached equipment, or other body accessory; and a functional device-attached light source attached to a functional device for augmented reality (AR), virtual reality (VR), or mixed reality (MR) (including goggles, a headset, and other functional devices).

In the first to third light radiation systems, the one or two or more light sources are regulated in orientation or position manually or automatically.

In the third light radiation system, a received effective spectral irradiance (W/m²/nm) found from a product of the spectral data of the light and the data of the OPN5 absorption spectrum, on a per wavelength basis, is calculated.

In the third light radiation system, a reference effective spectral irradiance (W/m²/nm) found from a product of a spectral irradiance (W/m²/nm) of each wavelength in a sunlight spectrum defined by AM1.5G and the OPN5 absorption spectrum, on a per wavelength basis, is calculated and compared with the received effective spectral irradiance (W/m²/nm).

In the third light radiation system, the spectral data of the light is compared in all or part of a wavelength region of the OPN5 absorption spectrum. In this application, "all" refers to a wavelength region within a range of 280 to 500 nm, and "part" refers to a wavelength region specified as desired from the entire wavelength region, such as a wavelength region within a range of 360 to 400 nm or 370 to 390 nm, for example.

In the third light radiation system, during comparison in the wavelength region, the received effective spectral irradiance (W/m²/nm) is integrated in the wavelength region to obtain a received effective irradiance (W/m²), the reference effective spectral irradiance (W/m²/nm) is similarly integrated in the same wavelength region to obtain a reference effective irradiance (W/m²), and the received effective irradiance and the reference effective irradiance thus obtained are compared. With this comparison, the third light radiation system is advantageous to quantitatively evaluating changes in the body that occur by the absorption of light by OPN5 present in the retina of the person.

In the third light radiation system, a received effective dose (J/m²) obtained by multiplying the received effective irradiance (W/m²) by the radiation duration (seconds) and a received reference effective dose (J/m²) obtained by multiplying the reference effective irradiance (W/m²) by the radiation duration (seconds) are compared. With this comparison, the third light radiation system is advantageous to quantitatively evaluating the changes in the body that occur by the absorption of light by OPN5 present in the retina of the person.

In the third light radiation system, the measurement is performed by a measurement unit, and the measurement unit is provided to eyeglasses, an earphone, a wristwatch, a chest badge, or other article worn on a body, worn on the body itself, or attached to an installation near or around the body, on the basis of a form of each of the one or two or more light sources that radiate the light.

(4) A light radiation method according to the present invention comprises radiating, to an irradiated subject, light having a wavelength in a wavelength region overlapping a peak wavelength of an OPN5 absorption spectrum from one or two or more light sources that radiate the light, comparing spectral data of the light obtained by receiving the radiated light and data of the OPN5 absorption spectrum, and regulating radiation conditions of the light (one or two or more conditions selected from an installation position, a radiation direction of the light, a wavelength range of the light, an irradiance of the light, a radiation time frame of the light, and a radiation duration of the light of each of the one or two or more light sources) on the basis of results of the comparison. This invention is an operating means for regulating the conditions for radiating the light, and the light to be radiated is regulated on the basis of a comparison between the spectral data of the received light and the OPN5 absorption spectral data, and thus the operating means is advantageous to the activation of OPN5 present in the retina of the person who is the irradiated subject.

This light radiation method further comprises a light measuring means for measuring the spectral data of the light and comparing the spectral data of the light and the data of the OPN5 absorption spectrum. A received effective spectral irradiance (W/m²/nm) found from a product of the spectral data of the light and the data of the OPN5 absorption spectrum, on a per wavelength basis, is calculated.

(5) A light measurement method according to the present invention comprises measuring spectral data of light having a wavelength in a wavelength region overlapping a peak wavelength of an OPN5 absorption spectrum, comparing the measured spectral data of the light and data of the OPN5 absorption spectrum stored in advance, and displaying results of the comparison on a display device or outputting the results as data.

According to this invention, the spectral data of the received light is measured and the measured spectral data of the light and known OPN5 absorption spectral data stored in advance are compared, and thus the obtained comparison results are evaluated, making it possible to help clarify a relationship between light radiation and visual functions and non-visual functions (including a relationship with myopia progression suppression).

In this light measurement method, the comparison is performed by a calculation unit, and a received effective spectral irradiance (W/m²/nm) found from a product of the spectral data of the light and the data of the OPN5 absorption spectrum, on a per wavelength basis, is calculated in the calculation unit.

In this light measurement method, a reference effective spectral irradiance (W/m²/nm) found from a product of a spectral irradiance (W/m²/nm) of a sunlight spectrum defined by AM1.5G and the OPN5 absorption spectrum, on a per wavelength basis, is calculated and compared with the received effective spectral irradiance (W/m²/nm).

According to this invention, it is possible to conduct a generalized evaluation of a degree (for example, percentage: %) of the actual received effective spectral irradiance relative to the common reference effective spectral irradiance, regardless of a measurement region or a measurement time. As a result, it is possible to utilize the measurement method in an objective evaluation of whether light radiation is related to physical effects on the basis of the comparison results.

In this light measurement method, the spectral data of the light is compared in all or part of a wavelength region of the OPN5 absorption spectrum.

According to this invention, even in a case in which the received light includes various spectral data, a comparison is conducted with all or part of the wavelength region of the OPN5 absorption spectrum, making it possible to apply the method to an evaluation in which the wavelength region of the light is specified using a filter or the like. As a result, the method can be utilized as a measurement method of clearly defining whether the physical effects changed by the light radiation are related to the received effective spectral irradiance across the entire wavelength region of the OPN5 absorption spectrum, related to the received effective spectral irradiance in a specific wavelength region of the OPN5 absorption spectrum, or the like. It should be noted that, for example, as a method of specifying a wavelength region of light by using a filter or the like, it is also possible to specify a specific wavelength region such as that of violet light or the like having the wavelength region of 360 to 400 nm, for example, and thus utilize the method in verification of detailed effects specifying a wavelength region and the like as well. In this application, "all" refers to a wavelength region within a range of 280 to 500 nm, and "part" refers to a wavelength region specified as desired from the entire wavelength region, such as a wavelength region within a range of 360 to 400 nm or 370 to 390 nm, for example.

In this light measurement method, during comparison in the wavelength region, the received effective spectral irradiance is integrated in the wavelength region to obtain a received effective irradiance (W/m²), the reference effective spectral irradiance is similarly integrated in the same wavelength region to obtain a reference effective irradiance (W/m²), and the received effective irradiance and the reference effective irradiance thus obtained are compared.

In this light measurement method, a received effective dose (J/m²) obtained by multiplying the received effective irradiance (W/m²) by a radiation duration (seconds) and a received reference effective dose (J/m²) obtained by multiplying the reference effective irradiance (W/m²) by the radiation duration (seconds) are compared. With this comparison, the light measurement method is advantageous to quantitatively evaluating the changes in the body that occur by the absorption of light by OPN5 present in the retina of the person.

In this light measurement method, the measurement is performed by a measurement unit, and the measurement unit is provided to eyeglasses, an earphone, a wristwatch, a chest badge, or other article worn on a body, worn on the body itself, or attached to an installation near or around the body, on the basis of a form of each of the one or two or more light sources that radiate the light. According to this invention, attachment in the above-described mode is selected and performed as desired on the basis of an installation form (light source form, light source installation position, and the like) of the light source that radiates the light, and thus is not likely to interfere with daily life and makes it easy to measure the spectral data of the light received by the eyes and the body.

(6) A light measurement device according to the present invention comprises a measurement unit that measures spectral data of light having a wavelength in a wavelength region overlapping a peak wavelength of an OPN5 absorption spectrum, compares the spectral data of the light measured by the measurement unit and data of the OPN5 absorption spectrum stored in advance, and displays results of the comparison on a display device or outputs the results as data.

This invention is a device that executes the light measurement method of the above-described (5) and, among the measurement unit, the calculation unit, and the output unit constituting the device, at least the measurement unit is built into the light measurement device, but the calculation unit and the output unit may be built into the light measurement device or may not be built into the device but rather provided in an external device or external equipment.

### Effect of the Invention

According to a first light radiation system of the present invention, each light source is regulated, making it possible to radiate light having a wavelength in a wavelength region overlapping a peak wavelength of an OPN5 absorption spectrum to an irradiated subject. As a result, the first light radiation system is advantageous to activation of OPN5 present in a retina of a person who is the irradiated subject.

According to a second light radiation system of the present invention, it is possible to radiate light in which a received effective spectral irradiance to be calculated is 0.2% or more of a reference effective spectral irradiance to the irradiated subject. As a result, the second light radiation system is advantageous to the activation of OPN5 present in the retina of the person who is the irradiated subject.

According to a third light radiation system of the present invention, an installation position, a radiation direction of the light, a wavelength range of the light, an irradiance, a radiation time frame, and a radiation duration of each light source are regulated on the basis of the results obtained through comparison by a light measuring means. As a result, the third light radiation system is advantageous to the activation of OPN5 present in the retina of the person who is the irradiated subject.

According to a light radiation method of the present invention, the light to be radiated is regulated on the basis of a comparison between spectral data of the light obtained by receiving the radiated light and OPN5 absorption spectral data, and thus the light radiation method is advantageous to the activation of OPN5 present in the retina of the person who is the irradiated subject.

According to a light measurement method and a light measurement device of the present invention, the spectral data of the received light is measured and the measured spectral data of the light and the known OPN5 absorption spectral data stored in advance are compared, and thus the obtained comparison results are evaluated, making it possible to expect clarification of the relationship between light radiation and visual functions and non-visual functions (including the relationship with myopia progression suppression). Such obtained comparison results can be utilized as basic data for evaluating the relationship between received light and OPN5 activation, particularly can be calculated and quantified on the basis of a degree of overlap between the measured light spectral data and the known OPN5 absorption spectral data, and thus can be utilized for objective evaluation of whether light radiation is related to physical effects.

### BRIEF DESCRIPTION OF THE DRAWINGS

Fig. 1 shows an absorbance (dimensionless) of an OPN5 absorption spectrum.
Fig. 2 shows absorption spectra of OPN5, OPN4, rhodopsin (Rh), and three photopsins (S, M, L).
Fig. 3 shows a standard photopia relative luminous efficiency (upper graph) and a color of light (lower chart) at each wavelength.
Fig. 4 shows an example of a violet light LED having a radiation wavelength range of 360 to 400 nm.
Fig. 5 (A) shows an example of a light source that radiates light having a wavelength within a wide wavelength range exceeding 400 nm, Fig. 5 (B) shows an example of a light source in which a wavelength range of the radiated light is restricted to 360 to 400 nm by a shielding filter, and Fig. 5 (C) is an explanatory view showing overlap between the restricted radiated light in the above-described Fig. 5 (B) and the OPN5 absorption spectrum.
Fig. 6 illustrates an installation example of the light sources in a large office space.
Fig. 7 illustrates an installation example of the light sources in a home or an office.
Figs. 8 (A) and 8 (B) illustrate installation examples of a desktop light source on a desk and a personal-computer-installed light source.
Fig. 9 (A) shows the absorbance (dimensionless) of the OPN5 absorption spectrum, Fig. 9 (B) shows a spectral irradiance (intensity received at an eye position) of a violet light LED having a peak at 375 nm, and Fig. 9 (C) shows a received effective spectral irradiance found from a product of the two at each wavelength.
Fig. 10 (A) shows the absorbance (dimensionless) of the OPN5 absorption spectrum, Fig. 10 (B) shows a spectral irradiance (intensity received at the eye position) of a wide-area light source emitting light at 300 nm or greater, and Fig. 10 (C) shows a received effective spectral irradiance found from a product of the two at each wavelength.
Fig. 11 (A) shows the absorbance (dimensionless) of the OPN5 absorption spectrum, Fig. 11 (B) shows a spectral irradiance of a sunlight spectrum defined by AM1.5G, and Fig. 11 (C) shows a reference effective spectral irradiance found from a product of the two at each wavelength.
Fig. 12 is a graph contrasting the reference effective spectral irradiance (reference sign b) shown in Fig. 11 (C) and the received effective spectral irradiance (reference sign a) shown in Fig. 9 (C).
Fig. 13 is a schematic diagram illustrating an example of a light measurement method and a light measurement device.

### Embodiments of the Invention

Hereinafter, a light radiation system, a light radiation method, a light measurement method, and a light measurement device according to the present invention will be described with reference to the drawings. The present invention is not limited to the embodiments below, and can be modified to various modes as long as the gist of this application is included.
(1) A light radiation system (first light radiation system) according to the present invention includes one or two or more light sources that radiate light having a wavelength in a wavelength region overlapping a peak wavelength of an OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated.
(2) A light radiation system (second light radiation system) according to the present invention includes one or two or more light sources that radiate light having a wavelength in a wavelength region overlapping a peak wavelength of an OPN5 absorption spectrum. A spectrum of the light received by an irradiated subject is regulated so that a received effective spectral irradiance (W/m²/nm) calculated as a product of a spectral irradiance (W/m²/nm) of each wavelength in a wavelength region of 280 nm to 500 nm of the light and a relative absorbance of data of the OPN5 absorption spectrum, on a per wavelength basis, is 0.2% or more of a reference effective spectral irradiance (W/m²/nm) calculated as a product of a spectral irradiance (W/m²/nm) of a sunlight spectrum defined by AM1.5G and a relative absorbance of the OPN5 absorption spectrum, on a per wavelength basis.
(3) A light radiation system (third light radiation system) according to the present invention includes a light radiating means including one or two or more light sources that radiate light having a wavelength in a wavelength region overlapping a peak wavelength of an OPN5 absorption spectrum, and a light measuring means for measuring spectral data of the light and comparing the spectral data of the light and data of the OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated on the basis of results obtained by the comparison.
(4) A light radiation method according to the present invention includes radiating, to an irradiated subject, light having a wavelength in a wavelength region overlapping a peak wavelength of an OPN5 absorption spectrum from one or two or more light sources that radiate the light, comparing spectral data of the light obtained by receiving the radiated light and data of the OPN5 absorption spectrum, and regulating radiation conditions of the light (one or two or more conditions selected from an installation position, a radiation direction of the light, a wavelength range of the light, an irradiance of the light, a radiation time frame of the light, and a radiation duration of the light of each of the one or two or more light sources) on the basis of results of the comparison.
(5) A light measurement method according to the present invention includes measuring spectral data of light having a wavelength in a wavelength region overlapping a peak wavelength of an OPN5 absorption spectrum, comparing the measured spectral data of the light and data of the OPN5 absorption spectrum stored in advance, and displaying results of the comparison on a display device or outputting the results as data.
(6) A light measurement device according to the present invention includes a measurement unit that measures spectral data of light having a wavelength in a wavelength region overlapping a peak wavelength of an OPN5 absorption spectrum, compares the spectral data of the light measured by the measurement unit and data of the OPN5 absorption spectrum stored in advance, and displays results of the comparison on a display device or outputs the results as data.

### [Components]

Hereinafter, components of each aspect of the above-described invention will be described in detail. It should be noted that each component is related to the respective aspects of the present invention and thus, in the following, will be described in order thereof and not in the order of the aspects.

### [OPN5, OPN5 Absorption Spectrum]

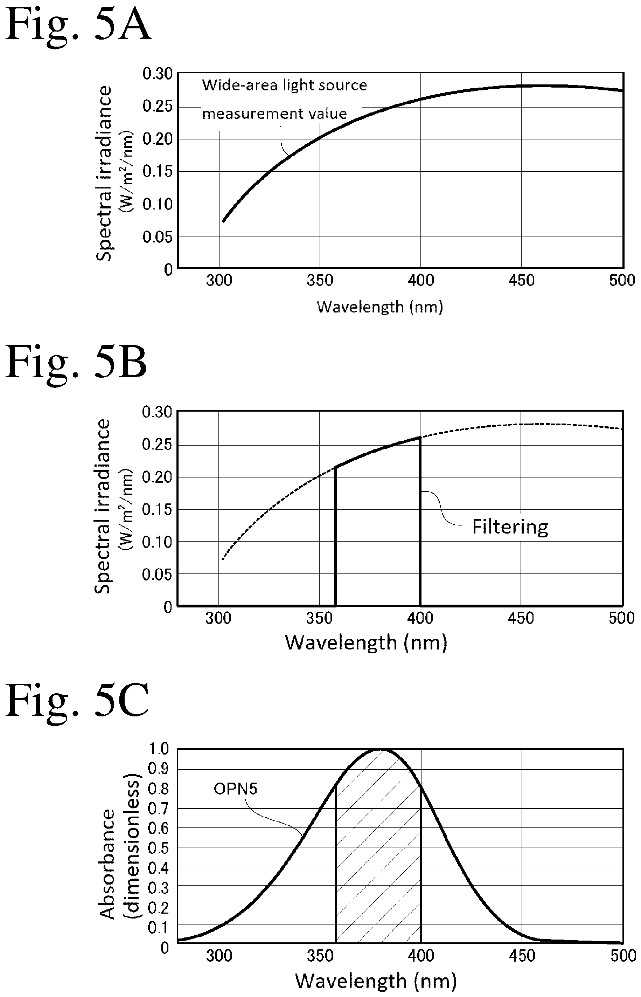

Opsin 5 (OPN5) is a photoreceptor protein and is present in the human retina. As shown in Fig. 1, OPN5 exhibits an absorption spectrum having a peak at 380 nm. In the present invention, the "OPN5 absorption spectrum" is defined as the absorption spectrum of OPN5 shown in Fig. 1. This Fig. 1 is cited from Fig. 5A in "Kojima D, Mori S, Torii M, Wada A, Morishita R, et al. (2011), UV-Sensitive Photoreceptor Protein OPN5 in Humans and Mice. PLoS ONE 6 (10), e26388. doi: 10.1371/journal. pone. 0026388" and is understood as standard by those skilled in the art at the time of the filing of this application. Further, it is clearly specified in the cited document and understood by those skilled in the art that this OPN5 absorption spectrum has an absorption peak at 380 nm. Through radiation of light in the present invention, activation of OPN5 present in the retina of the person who is an irradiated subject can be expected, and improvement of visual functions and non-visual functions, realization of myopia progression suppression and the like, and the pursuit and development of research in these areas can be expected. It should be noted that Fig. 2 shows absorption spectra of OPN5, OPN4, rhodopsin (Rh), and three photopsins (S, M, L). These are also described in Fig. 5A of the cited document by Kojima et al. described above. OPN5 is positioned in a lowest wavelength region among these.

The OPN5 absorption spectrum is an absorption spectrum in which, given 1 as the absorbance at the peak of 380 nm, a wavelength range corresponding to an absorbance of 0.1 or higher is within a range of approximately 300 nm to 440 nm, a wavelength range corresponding to an absorbance of 0.5 or higher is within a range of approximately 340 nm to 420 nm, a wavelength range corresponding to an absorbance of 0.7 or higher is within a range of approximately 350 nm to 410 nm, and a wavelength range corresponding to an absorbance of 0.8 or higher is within a range of approximately 360 nm to 400 nm, as shown in Fig. 1. For OPN5 having such absorbance properties, light exhibiting a high absorbance of, for example, 0.5 or higher, 0.7 or higher, or 0.8 or higher is preferably radiated from the viewpoint of OPN5 activation.

### [Light Source, Light Radiating Means, Light Radiation Method]

### <Light>

In the present invention, the light radiated from the light source or the light received by the irradiated subject includes light having a wavelength in a wavelength region overlapping the peak wavelength of the OPN5 absorption spectrum. The peak wavelength of the OPN5 absorption spectrum is 380 nm, as described above. Here, "light having a wavelength in a wavelength region overlapping the peak wavelength" may be any light having a wavelength in a wavelength region including the peak wavelength (380 nm) of the OPN5 absorption spectrum shown in Fig. 1. This means, for example, that the light may be light having a wavelength in a wavelength region overlapping the wavelength region of the entire range of approximately 270 nm to 460 nm, which is the entire wavelength region shown in Fig. 1, or may be light having a wavelength in a wavelength region overlapping part of the wavelength region. As described above, "part of the wavelength region" means that the light need only include the peak wavelength (380nm), even without overlapping the wavelength region of the entire range of the OPN5 absorption spectrum (approximately 270 nm to 460 nm), such as, for example, the wavelength range in which the absorbance is 0.1 or higher (approximately 300 nm to 440 nm), the wavelength range in which the absorbance is 0.5 or higher (approximately 340 nm to 420 nm), the wavelength range in which the absorbance is 0.7 or higher (approximately 350 nm to 410 nm), or the wavelength range in which the absorbance is 0.8 or higher (approximately 360 nm to 400 nm).

It should be noted that the "light having a wavelength in a wavelength region including the peak wavelength (380 nm) of the OPN5 absorption spectrum" may be, for example, light including a wavelength range of ±20 nm of the peak wavelength (380 nm) of the OPN5 absorption spectrum or light partially overlapping the peak wavelength. This means that the radiated light is more preferably light having a peak within the wavelength range of 380 nm ±20 nm, but may be light having a wavelength in a wavelength region that does not have a peak, but emits light within the range. Further, "light including" is light radiating wavelengths of 360 to 400 nm including 380 nm ±20 nm, and "light partially overlapping" means light at least overlapping 380 nm, for example, including a wavelength region of 370 to 400 nm, 360 to 390 nm, 365 to 395 nm, or the like. It should be noted that the present inventors refer to light having a wavelength within the wavelength range of 360 nm to 400 nm as violet light, and thus, hereinafter, the light having a wavelength within this wavelength range may be described as violet light.

"Radiated" means that the light emitted from the light source is radiated to the person who is the irradiated subject. A radiated mode may be a mode in which the light source is directed toward the irradiated subject who is the radiation target, and the light emitted from the light source is directly radiated to the irradiated subject; a mode in which the light emitted from the light source at a position in a field of view of the person who is the irradiated subject or at a position along the subject' s visual line is radiated to the irradiated subject; or a mode in which the light source is not directed toward the irradiated subject, and the light emitted from the light source is radiated as diffused light or reflected light to the irradiated subject. "Received light" refers to the light received by the person who is the irradiated subject. The irradiance and the absorption spectrum of the received light are measured by a measurement device attached to the person or a position around the person. It should be noted that, in this application, the irradiated subject, the person, and the eyes of the person have the same meaning and can be used interchangeably.

### (Wavelength range of light)

For humans, it is said that light having a wavelength of less than 315 nm has a negative effect on the eyes, and thus a lower limit of the wavelength of the light is preferably 340 nm, 350 nm, or 360 nm. The wavelength among these that serves as the lower limit depends on the irradiance of the light in the lower limit region. If the irradiance of the light near the lower limit is small, the wavelength may be less than 340 nm. However, preferably the light is within a lower limit range of 340 nm, 350 nm, or 360 nm, and more preferably the light is within a lower limit range of 350 nm or 360 nm. It should be noted that, in the OPN5 absorption spectrum shown in Fig. 1, the absorbance is 0.5 or higher within a wavelength range of approximately 340 nm to 420 nm, 0.7 or higher within a wavelength range of approximately 350 nm to 410 nm, and 0.8 or higher within a wavelength range of approximately 360 nm to 400 nm. Therefore, by adopting a light source that radiates light having a wavelength such as within a wavelength range of approximately 340 nm to 420 nm, within a wavelength range of approximately 350 nm to 410 nm, or within a wavelength range of approximately 360 nm to 400 nm, it is possible to contribute to the activation of OPN5.

The light is preferably light having an upper limit wavelength of 400 nm or less or 410 nm or less. Light having such a wavelength is light having a low relative luminous efficiency. Light having a low relative luminous efficiency is light that is not too bright. "Light that is not too bright" is light that is not bright for the person who is the irradiated subject. Even if such light is radiated, the person will not recognize or will find it difficult to recognize the brightness, and thus such light is advantageous to facilitating long-period radiation directed toward the person. "Radiation of light having a wavelength of 400 nm or less or 410 nm or less and having a low luminous efficiency" include cases in which the light is radiated from a light source that does not emit or substantially does not emit light exceeding 400 nm or exceeding 410 nm. As such a light source, violet light of 360 nm to 400 nm or light of 350 nm to 410 nm is particularly preferred. Further, even in a case in which the light is radiated from a light source including a filter that blocks light having a wavelength exceeding 400 nm or exceeding 410 nm or a case in which light having a wavelength exceeding 400 nm or exceeding 410 nm is emitted, it is possible to radiate light having a wavelength of 400 nm or less or 410 nm or less.

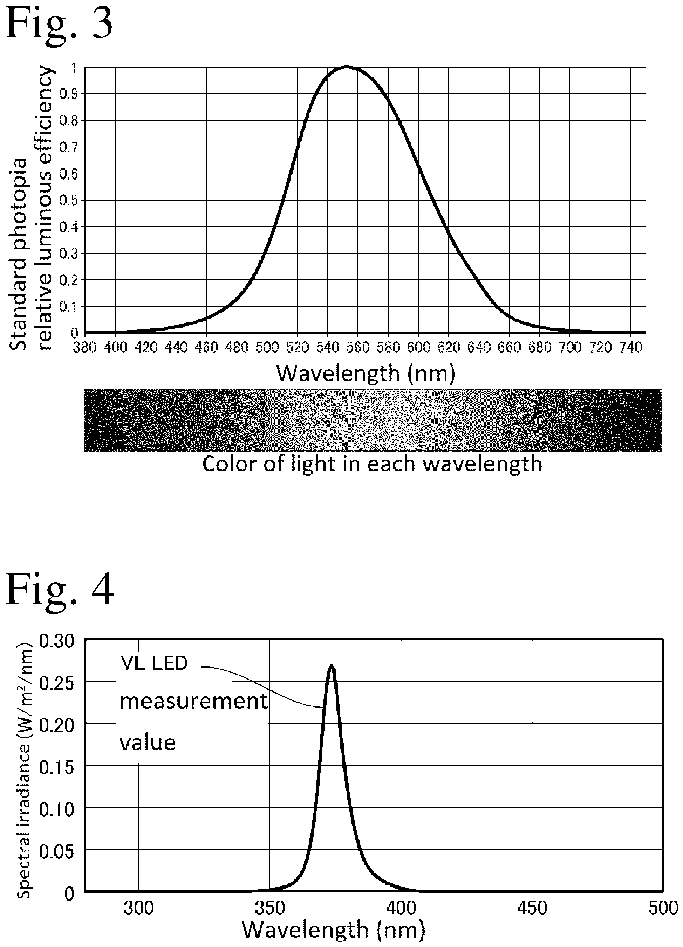

The relative luminous efficiency, according to "Change of Brightness of Colored Lights Measuring by the Direct Comparison Method" (Masanori Takase, Katsunori Okajima, Keiji Uchikawa, and Mitsuo Ikeda, Japanese Journal of Optics, Vol. 19, No. 3, 1990), for example, can be measured using methods such as a direct comparison method, a flicker photometry method, an iteration method, a minimally distinct border (MDB) method, and an absolute threshold method. The standard CIE relative luminous efficiency function is described as mainly determined using the flicker photometry method, and typically is evaluated using the direct comparison method and the flicker photometry method. It should be noted that the data for the relative luminous efficiency (spectral luminous efficiency) is listed in Appendix Table 8 of "https://elaws.e-gov.go.jp/download?law_unique_id=404M50000400080_20161001_000000000000000&file_t ype=PDF_H1." The relative luminous efficiency (relative luminous efficiency in a bright location) is standardized by light having a wavelength of 555 nm (green light) and, from this table, for example, a relative luminous efficiency of 0.01 or less corresponds to a wavelength region of less than 430 nm on the short wavelength side. Thus, humans perceive green light as the brightest, and the sensation of brightness decreases whether the wavelength increases or decreases from that point. The relative luminous efficiency is obtained by, given 1 as the brightness at a wavelength of 555 nm, expressing the brightness of other wavelengths having the same energy as comparison values. Fig. 3 shows a standard photopia relative luminous efficiency. There are individual differences in how people actually perceive light that is not bright, making it difficult to ascertain relative values on the vertical axis shown in Fig. 3. However, the light having a wavelength of 400 nm or less or 410 nm or less described above has a wavelength lower than the wavelength region of less than 430 nm, which has a relative luminous efficiency of 0.01 or less, and thus can be regarded as light having an even lower relative luminous efficiency and at least a relative luminous efficiency of less than 0.01. Light having a wavelength of 400 nm or less or 410 nm or less with a relative luminous efficiency of such an ultra-low value can be considered light that is not bright in comparison with light in other wavelength regions in which the relative luminous efficiency exceeds 0.01, regardless of individual differences. As a result, in a case in which light is radiated to a person without causing a sense of brightness, such light is advantageous to allowing both direct radiation and long-period radiation. It should be noted that, in this application, the irradiated subject, the person, and the eyes of the person have the same meaning and can be used interchangeably.

### <Light source>

The light source is not particularly limited as long as the light source radiates light having a wavelength within the wavelength range described above. "Radiates light having a wavelength within the wavelength range" means that the emission spectrum of the light source itself may be within the wavelength range described above (for example, light of 360 nm to 400 nm or 350 nm to 410 nm) or, even if the emission spectrum of the light source itself exceeds the upper limit of the wavelength range described above, may be within the wavelength range described above through a shielding filter. Further, as shown in Fig. 4, a light source that does not emit or substantially does not emit light having a wavelength exceeding 400 nm (for example, a violet light LED light source having a peak wavelength at 375 nm) may be adopted or, for a light source that radiates light having a wavelength within a wide wavelength range exceeding 400 nm as shown in Fig. 5 (A), a light source including a shielding filter shown in Fig. 5 (B) may be adopted. It should be noted that Fig. 5 (B) shows an example of a light source in which the wavelength range of the radiated light is restricted to 360 to 400 nm by the shielding filter, and Fig. 5 (C) is an explanatory view showing overlap between the light radiated from the light source and the OPN5 absorption spectrum.

For example, light of 360 nm to 400 nm or 350 nm to 410 nm overlaps the absorption spectrum of OPN5 having a peak at 380 nm, and has a low relative luminous efficiency and is not bright, and thus is advantageous to that, even when the light is radiated to the eyes of a person as direct light, as diffused light, or as reflected light, the light is relatively unobtrusive and can activate OPN5 present in the retina of the person, for example.

It should be noted that, in a case in which light is radiated toward the eyes of the person who is the irradiated subject, a light source that emits only light of 400 nm or less or 410 nm or less that is not bright is desirably used. However, in a case of a light source disposed so as to not radiate light toward the eyes of the person, the light source may include light having a wavelength exceeding 400 nm or exceeding 410 nm (white light and the like) along with the light of 400 nm or less or 410 nm or less, or may be obtained by combining a light source of 400 nm or less or 410 nm or less and a light source that emits light having other wavelengths.

The light emitted from the light source may be constant light or flickering light. In the case of flickering light, ongoing flickering light may be continuously applied, or may be applied at intervals of several seconds, tens of seconds, several minutes, or the like.

### <Installation form of light source>

One or two or more light sources are provided. Each light source is selected from a structure-attached light source attached to a floor (including underfoot), a wall, a ceiling (including a suspended ceiling), or other structure; an installation-attached light source attached to a desk, a table, a partition (divider, screen), a shelf, a desktop member, a personal computer (PC), a display, a PC keyboard, a television, an audio device, or other equipment or installation; a built-in or attached light source of a portable PC, a tablet, a smartphone, or other mobile terminal; a body-accessory-attached light source detachably mounted or attached to a vision correcting aid, eye protective equipment, face protective equipment, neck hanging accessory, ear hooking accessory, head-attached equipment, or other body accessory; and a functional device-attached light source attached to a functional device for augmented reality (AR), virtual reality (VR), or mixed reality (MR) (including goggles, a headset, and other functional devices). Each light source is, in accordance with a form thereof, built into or attached to a structure, a device, an installation, a mobile terminal, a body accessory, a functional device, or the like, and the light emitted from the light source can be radiated to the person as direct light, as diffused light, or as reflected light.

Such a light source form may be an installation-attached light source attached as is inside housing such as a private home, a public or shared building such as a school building, a government building, a company building, and a gymnasium, or in a structure covered by a roof but without walls, or may be an installation-attached light source attached to furniture or other installations. Further, the light source may be a portable light source that can be installed on a mobile terminal such as, for example, a portable personal computer, a tablet, or a smartphone that can be carried by a person. Furthermore, the light source may be a wearable light source attached to, for example, a vision correcting aid, eye protective equipment, face protective equipment, neck hanging accessory, ear hooking accessory, head-attached equipment, or the like that can be worn by a person. It should be noted that the wearable light source that can be worn by a person may be an eyeglasses-installed light source in which a light source is attached to eyeglasses that can be worn by a person, regardless of whether the eyeglasses have a vision correction function, an attachment light source attachable to and detachable from such eyeglasses, or may be a light source attached to goggle-type device for functional use, augmented reality (AR) use, virtual reality (VR) use, or mixed reality (MR) use.

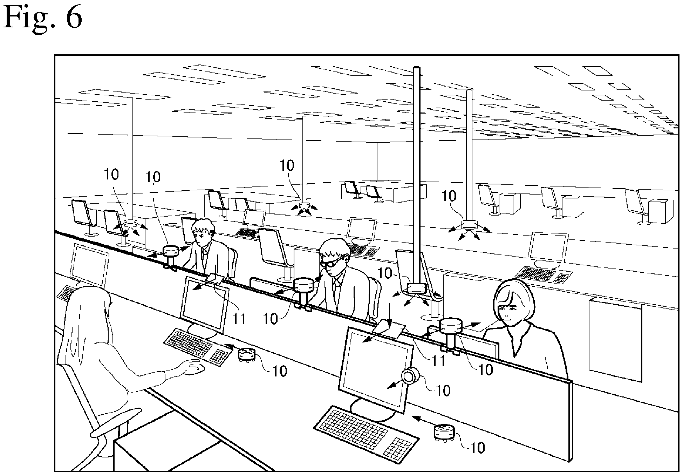

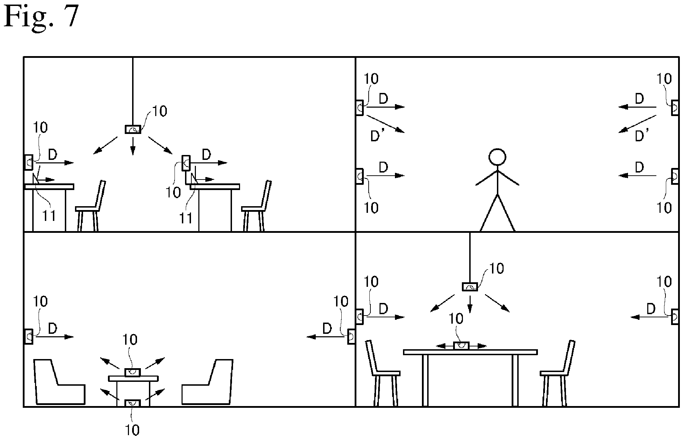

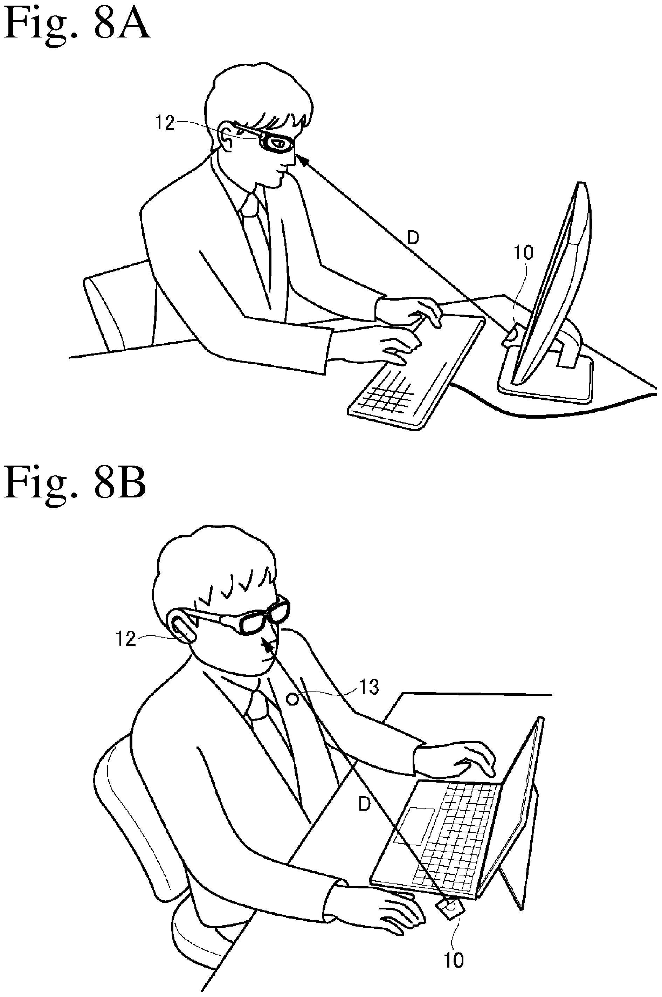

Fig. 6 to Fig. 8 illustrate installation examples of the light source. Fig. 6 is an installation example of the light sources in a large office space, Fig. 7 is an installation example of the light sources in a home or office, and Fig. 8 is an example of a desktop light source on a desk and a personal-computer-installed light source. Thus, the light source can be installed in various modes.

"One or two or more light sources" means one or two or more light sources selected from the various light sources described above (installation-type light source, body-accessory-type light source, functional device-installed light source). In a case of one light source, the light source is desirably disposed so that the light emitted from the light source is directed toward the eyes.

Regarding specific installation forms of the installation-type light source, as illustrated in Fig. 6 and Fig. 7, for example, in a case in which the direction of the visual line does not change much, such as a case in which the focus is on deskwork in an office or studying in a school, a study hall, or a home, an installation-type light source 10 is desirably disposed ahead in the visual line, with an orientation of the installation-type light source 10 regulated so that the light is emitted toward a direction D of the eyes. Further, in Fig. 6 and Fig. 7, the light source 10 is disposed at a position in the field of view or a position in the visual line direction, and may be disposed at such a position. Furthermore, even in a case in which the light source 10 is not directed toward the person, the light source 10 may be disposed at a position where the light can be radiated to the eyes (for example, a position outside the field of view or other than in the visual line direction) by utilizing a diffusing material or a reflecting material 11 (refer to Fig. 6 and Fig. 7). Thus, the one or two or more light sources 10 can be provided at various positions. Fig. 6 and Fig. 7 illustrate examples of the installation-type light source in which the light source 10 is mainly installed on a wall, suspended from the ceiling, and installed on a desk, a table, a floor (including underfoot), and the like, but the light source 10 may be provided to other structures, and provided to a partition (divider, screen), a shelf, a desktop member, or other installations. In addition, as illustrated in Fig. 8, the light source 10 is desirably provided, with an orientation of the installation-type light source 10 regulated, at a position in the field of view and a position in the visual line direction, such as, for example, a position on a frame part of a display or an extension thereof, or a position around a keyboard of a personal computer.

Further, as illustrated in Fig. 7, in a case in which the possibility exists that the direction of the visual line may change at work, at home, or the like, desirably two or more installation-type light sources 10 are disposed and the positions and the orientations of the light sources 10 are regulated so that the light is emitted in the direction of the eyes to the extent possible. For example, in a case in which the person often sits on a chair or a sofa, the installation-type light sources 10 are desirably disposed at eye height to the extent possible and installed at the height in directions of the eyes to the extent possible. Furthermore, similarly, in a case in which the person is often standing, the installation-type light sources 10 are desirably disposed at eye height to the extent possible and installed at the height in directions of the eyes to the extent possible. It should be noted that, in a case in which the person sits and stands, the installation-type light sources 10 are desirably installed so as to satisfy both conditions and installed at the respective heights in directions of the eyes to the extent possible.

For example, light such as light having a wavelength of about 400 nm or less that is not bright is typically light that is less likely to be reflected on walls, floors, and the like, and thus the light source is desirably disposed at a position that directly radiates the light toward the irradiated subject. On the other hand, even in a case in which the light is not radiated directly toward the irradiated subject, a) the light source can be disposed at a position in the field of view of the irradiated subject or a position in the visual line direction as viewed from the irradiated subject side, b) the light source can be disposed at a position that radiates the light to the irradiated subject as diffused light diffused by a diffusing material or, c) by providing a special reflecting material that reflects light having a wavelength of 400 nm or less as appropriate, the light source can be disposed at a position that radiates the light to the irradiated subject as reflected light reflected by such a reflecting material. For example, for deskwork, the light source is desirably installed at a position in a direction directly in front of the eyes, at a field-of-view position, or at a position in the visual line direction of the person who is the irradiated subject. However, even in a case in which installation at these positions is not possible or is difficult, preferably the diffusing material or the reflecting material is provided and the light is diffused or reflected by the diffusing material or the reflecting material, thereby radiating diffused light or reflected light to the eyes of the person who is the irradiated subject. Preferably, the light is radiated to the eyes of the person using one or two or more of such means in combination. The diffusing material can be selected from various diffusing materials on the basis of diffusion angle properties and transmittance properties. The reflecting material, with utilization of a special reflecting material that reflects the light described above that is less likely to be reflected as a fixed, movable, or portable type, has the advantage of increasing a degree of freedom in light source installation locations.

Preferably, the installation-type light sources have, in all cases, a mode of advance installation with the orientation of light in the direction of the eyes, or a mode of installation at positions in the field of view or positions in the visual line direction is preferred. Further, the installation orientation and position of each light source may be manually or automatically regulated, and the angle of the light emitted from each light source may be manually or automatically regulated. The orientation or position of each light source is manually or automatically regulated, making it possible to change the orientation and the position in which the light is radiated directly or by reflection toward the irradiated subject as desired. As a result, effective activation of OPN5 can be expected. "Regulated in orientation" means regulation by rotation in each three-dimensional direction of X, Y, and Z as desired, and "regulated in position" means regulation by movement in each of the three-dimensional directions of X, Y, and Z as desired. "Manual operation" can be exemplified by an angle adjustment adapter or the like capable of finely adjusting an attachment stand of the light source in a vertical direction, a vertically inclined direction, a horizontal rotation direction, and the like as with a camera adjustment adapter, for example. "Automatic operation" can be exemplified by a mechanism capable of automatically adjusting the angle adjustment adapter described above or the like by a motor drive. It should be noted that, in the case of manual operation, an operator performs the adjustment manually. However, in the case of automatic operation, examples include a case in which the angle adjustment adapter is provided with a charge-coupled device (CCD) camera or the like to detect the position of the irradiated subject and then automatically adjust by the motor drive. Further, the light source provided with a tracking system that detects the eyes or movement of the person and radiates the light is also effective. Using a light source that can be caused to swing in various directions or that has wide directional angles of emission is also effective. It should be noted that, in a case in which a diffusing material or a reflecting material is utilized, regulations such as changing the light source position may be made, or regulations such as changing a type of the diffusing material or an installation angle of the reflecting material may be made.

Examples of mobile terminal-installed light sources include light sources that can be attached to a mobile terminal such as a portable personal computer, a tablet, and a smartphone, but such a light source may be one that can be installed on mobile terminals other than these. Similar to the installation-type light source, the mobile terminal-installed light source is preferably installed or the like in advance so that the orientation of the light is in the direction of the eyes. Further, similar to the above-described installation-type light source, the installation position of the light source may be changed manually or automatically, and the angle of the light emitted from the light source may be changed manually or automatically. As described above, the light source provided with a tracking system is also effective, and using a light source that can be caused to swing in various directions or that has wide directional angles of emission is also effective.

The body-accessory-type light source includes an attachment light source or the like attachable to or detachable from a vision correcting aid, eye protective equipment, face protective equipment, neck hanging accessory, ear hooking accessory, head-attached equipment, or the like, but may be a light source other than these as long as the light source can be worn on the body. Similar to the above-described light sources, the body-accessory-type light source is also preferably installed or the like in advance so that the orientation of the light is in the direction of the eyes. It should be noted that the body-accessory-type light source is positioned at a short distance from the eyes and thus, in comparison with the light sources described above, there is no need to change the installation position. However, the installation position may be changed manually or automatically as necessary, and the angle of the light emitted from the light source may be changed manually or automatically.

The functional device-installed light source is a light source attached to a functional device (goggles, headset, or the like) for AR, VR, MR, or the like, but may be a light source that can be installed on equipment other than these. The functional device-installed light source, similar to the above-described body-accessory-type light source, is positioned at a short distance from the eyes and thus, in comparison with the light sources described above, there is no need to change the installation position. However, the installation position may be changed manually or automatically as necessary, and the angle of the light emitted from the light source may be changed manually or automatically.

### <Radiation form>

The light emitted from the light source is radiated toward a person, particularly preferably toward the eyes. "Radiated" refers to the light emitted from the light source being radiated to the person, and the light emitted from the light source may be radiated directly to the person by directing the light source toward the person, the light emitted from the light source at a position in the field of view or a position in the visual line direction may be radiated or, even without directing the light source toward the person, the light emitted from the light source may be radiated as diffused light or reflected light. "Directly or by reflection" means that the light may be radiated directly from the light source toward the person (preferably the eyes) or may be radiated by reflection by a reflecting material. Further, "radiated directly" also includes the meaning in which the light is the diffused light diffused by the diffusing material and the diffused light is radiated to the eyes. Furthermore, as described above, the orientation or position of the light source may be regulated manually or automatically, making it possible to change, as desired, the mode in which the light is efficiently radiated directly or by reflection toward the person.

In such a radiation form of the light, preferably (i) in a case in which the light source is provided at a position where the light is radiated toward the irradiated subject, the irradiance of the light is regulated in accordance with a distance and/or an angle from the light source to the irradiated subject, (ii) in a case in which the light source is provided at a position in the field of view of the irradiated subject or a position in the visual line direction from the irradiated subject side, the irradiance of the light is regulated in accordance with the distance and/or the angle from the light source to the irradiated subject, (iii) in a case in which the light source is provided at a position where the light is radiated to the irradiated subject as the diffused light diffused by the diffusing material, the irradiance of the light is regulated in accordance with a distance and/or an angle from the light source and the diffusing material to the irradiated subject, or a diffusion angle and/or a transmittance of the diffusing material, and (iv) in a case in which the light source is provided at a position where the light is radiated to the irradiated subject as the reflected light reflected by the reflecting material, the irradiance of the light is regulated in accordance with a distance and/or an angle from the light source and the reflecting material to the irradiated subject, or a reflection angle and/or a reflectance of the reflecting material. The irradiance of the light is regulated by each of such (i) to (iv), making it possible to deliver light having the irradiance necessary to activate OPN5 to the person.

### (Reflecting material)

The reflecting material is a member that reflects the light radiated in the present invention in a specific direction. For example, light having a wavelength of 400 nm or less or 410 nm or less is not reflected by a reflecting material that reflects normal white light. Therefore, a special reflecting material that reflects light having a wavelength of 400 nm or less or 410 nm or less is provided, making it possible to reflect the light by the reflecting material and radiate the light toward the person even in a case in which such light is not directly radiated toward the person or cannot be directly radiated due to installation constraints.

In a case in which the light source cannot be disposed at a position that allows the light to be directly radiated toward the eyes due to installation circumstances of the light source, the special reflecting material described above is installed at an appropriate position, making it possible to radiate the reflected light toward the eyes. For example, for the deskwork illustrated in Fig. 6, the light source 10 is desirably installed in front of the eyes. However, in a case in which the light source cannot be installed in front of the eyes, preferably the reflecting material 11 is provided in front of the eyes and the light is reflected and directed toward the eyes by the reflecting material 11. The reflecting material denoted by reference sign 11 in Fig. 6 is an example of a reflecting material that reflects light from a light source suspended from the ceiling toward the eyes. With utilization of a fixed, movable, or portable reflecting material, for example, the reflecting material has the advantage of increasing the degree of freedom in the light source installation location. Thus, the light source is desirably installed at a position where the light emitted from the light source is directly radiated to the eyes, but in other cases, the reflecting material is preferably utilized to radiate the light toward the eyes.

The reflecting material can be installed by applying resist ink onto a plate material or a wall. Specifically, examples include a high ultraviolet reflective white resist ink manufactured by Okamoto Glass Co., Ltd, and the like. In an experiment in which a reflecting material formed by applying the white resist ink at a thickness of 20 µm onto a glass substrate is irradiated with NSPUS10CS (shell-type LED, peak wavelength: 375 nm) manufactured by Nichia Corporation at an irradiance of 69 µm/cm² and a distance of 10 cm from the reflecting material, the reflectance can be increased by approximately 2.9 times.

### (Directionality)

Regarding the directionality of the light, desirably the light source is installed and the diffusing material or the reflecting material is installed so that the light reaches an orientation of the face (eye line) in particular. A height of the light source is also an important factor. For example, preferably the light source is installed at a position based on a behavioral pattern in the office or the home, and is regulated in height and direction. As a means for increasing the directionality, preferably the light source is installed so that the light emitted from the light source is directed directly toward the eyes, or the light source is installed at a position in the field of view or a position in the visual line direction so that the light emitted from the light source is readily directed toward the eyes. For example, in a case in which variation in a visual-line axis is relatively small (for example, during deskwork such as illustrated in Fig. 6 and Fig. 8), the light source is preferably installed at a position where the light is radiated at an angle of 30° or less with respect to the virtual visual-line axis of the person. Thereby, it is possible to radiate the light to the eyes of the person who is the irradiated subject.

It should be noted that diffusing materials with distinctive characteristics in terms of diffusion angle properties and transmittance properties are commercially available, making it possible to select from various diffusing materials on the basis of such properties.

### [Light Measurement Method and Light Measuring Means]

Next, the light measurement method and the light measuring means will be described. The light measurement method and the light measuring means are different in categorical expression, but have the same meaning and content, and thus hereinafter may simply be referred to as the light measuring means. This light measuring means may include the light radiation system according to the present invention.

In the present invention, the light measuring means for measuring the spectral data of the light irradiated from the light source and received by the person who is the irradiated subject and comparing the spectral data of the light with OPN5 absorption spectral data is preferably provided. Thus, with the light measuring means, it is possible to measure the spectral data of the received light and compare the measured spectral data of the light with known OPN5 absorption spectral data stored in advance and thus, by examining the obtained comparison results (also called comparison data), help clarify the relationship between light radiation and visual functions and non-visual functions (including a relationship with myopia progression suppression). The obtained comparison results can be utilized as basic data for examining and evaluating the relationship between the received light and the activation of OPN5, particularly can be compared and quantified on the basis of a degree of overlap between the measured light spectral data and the known OPN5 absorption spectral data, and thus can be utilized for objective evaluation of whether light radiation is related to physical effects. Through such utilization, for example, the light measuring means can be utilized for evaluating what wavelength range of light, irradiance level, radiation time frame, and radiation duration are effective in relation to the physical effects of OPN5.

Further, on the basis of the obtained comparison results, in a case in which the light cannot be sufficiently radiated to the irradiated subject, it is possible to regulate one or two or more conditions selected from the installation position, the radiation direction of the light, the wavelength range of the light, the irradiance of the light, the radiation time frame of the light, and the radiation duration of the light of each light source. As a result, the light measuring means is advantageous to the activation of OPN5 present in the retina of the person who is the irradiated subject. It should be noted that the "spectral data of the light" is spectral data of the light measured by a measurement unit provided at the position of the eyes of the irradiated subject or at a position around the eyes. The light measuring means constituting the system may be disposed integrally within the system with the light radiating means described above, or may be disposed at a separate location as a separate body through a communicating means. Furthermore, the measurements carried out by the light measuring means are performed within the system, but the comparison may be conducted by displaying the results at a separate location as a separate body through the communicating means or by performing comparison calculations. The light measuring means as a separate body may be a measuring means that performs measurement and comparison at each time of measurement and comparison, and indicates the results for regulating the light radiation. In a case in which the comparison is performed by providing a calculation unit, the measurement unit and the calculation unit may be always disposed within the system to perform continuous or on-demand measurement and comparison, or may be disposed within the system when needed to perform on-demand measurement and comparison.

It should be noted that the "light" in the "spectral data of the received light" means the spectral data of light including not only the light radiated from the light source but also all light including light from sources other than the light source to be measured, but indoors is mainly the spectral data of the light radiated from the light source. Further, this light measuring means is preferably capable of accurately measuring the light entering the eyes of the person and, in particular, is preferably one worn on the person or attached to a member worn on the person. The comparison is performed by the calculation unit, but may be performed by a calculation unit built into the light measurement method, or may be performed by transmitting to an external calculation unit not built into the light measurement method.

The comparison results can be displayed on a display device or output as data. A portion that performs such a display or output may be referred to as an output unit. It should be noted that the portion can also be called a display unit. This output unit, similar to the calculation unit described above, may be disposed within the same system or device, or may be disposed at a separate location as a separate body through the communicating means. The output unit as a separate body may be one that outputs (displays or outputs data related to) the comparison results, may be one always disposed within the system to perform continuous or on-demand output, or may be one disposed within the system when necessary to perform on-demand output.

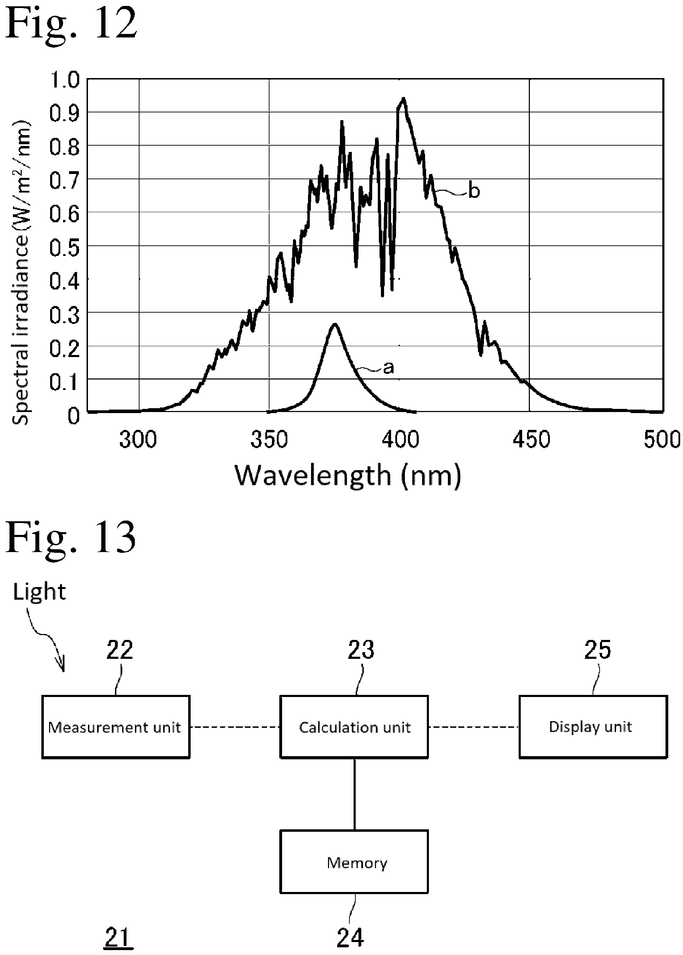

As an example of the light measuring means, as illustrated in Fig. 13, distinctive characteristics of a light measuring means 21 include a measurement unit 22 that measures received light spectral data, and a calculation unit 23 that compares the light spectral data and the OPN5 absorption spectral data. It should be noted that, as illustrated in Fig. 13, a memory 24 and a display unit 25 may be included. The memory 24 stores the OPN5 absorption spectral data and the like. The display unit 25 displays the comparison results and is also referred to as an output unit. The term "comparison" in relation to the calculation unit 23 is used with the meaning of including a case in which the data is simply compared and displayed or output, and a case in which the compared data is calculated and displayed or output.

This light measuring means 21 can measure the received light spectral data by the measurement unit 22, and compare the measured light spectral data with the OPN5 absorption spectral data by the internal or external calculation unit 23. Thus, the obtained comparison results are examined, making it possible to help clarify the relationship between light radiation and visual functions and non-visual functions (including the relationship with myopia progression suppression). In particular, the degree of overlap between the measured light spectral data and the OPN5 absorption spectral data stored in advance or the like is quantified through comparison calculation, making it possible to utilize the results for objective evaluation of the physical effects through OPN5. Through such utilization, for example, the light measuring means can be utilized for evaluating what wavelength range of light, irradiance level, radiation time frame, and radiation duration are effective in relation to the physical effects of OPN5. It should be noted that the light in the "received light spectral data" is not limited to light from the light source in the light radiation method described above, and means the light spectral data including that of all measured light. Further, this light measurement method is preferably capable of accurately measuring the light entering the eyes of the person and, in particular, is preferably one worn on the person or attached to a member worn on the person.

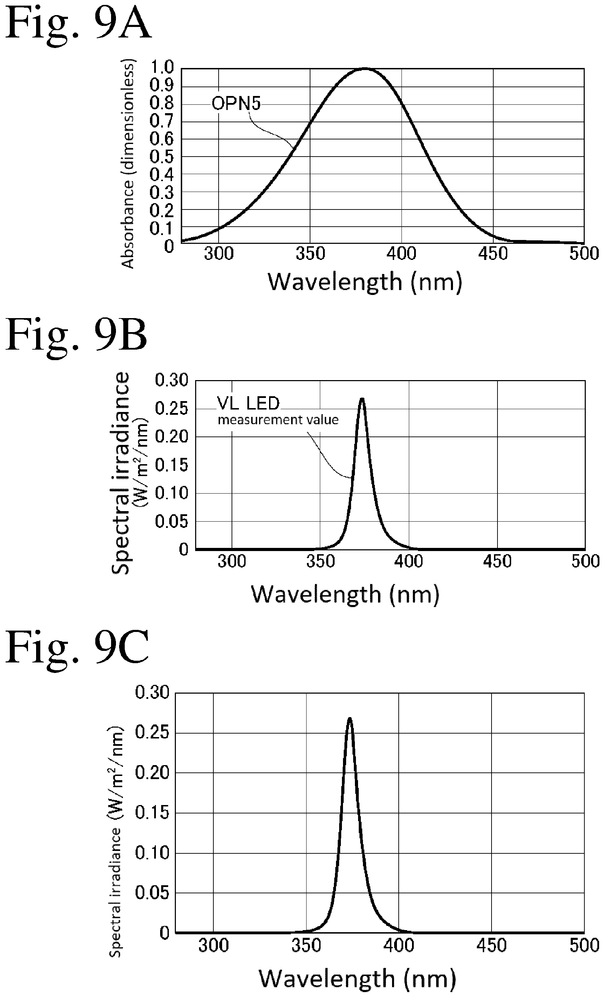

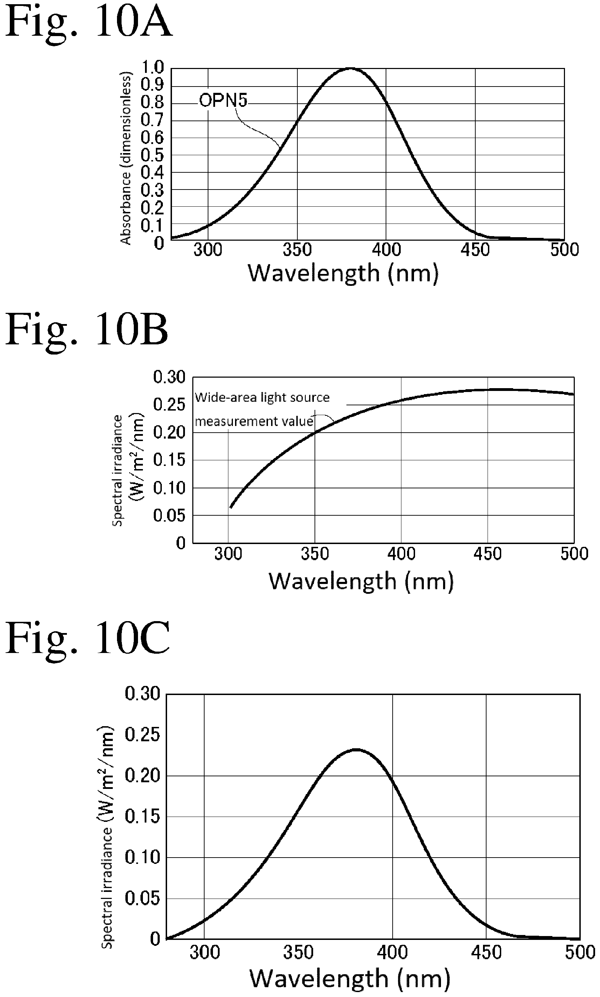

As shown in Fig. 9 and Fig. 10, the calculation unit calculates a product of the light spectral data at each wavelength and the OPN5 absorption spectral data, on a per wavelength basis, as the received effective spectral irradiance (W/m²/nm). Thereby, it is possible to use the received effective spectral irradiance (W/m²/nm) found from the product of both at each wavelength as evaluation data, and thus utilize the obtained evaluation data as a measurement device for clearly defining the objective relationship between the obtained evaluation data and the physical effects through OPN5.

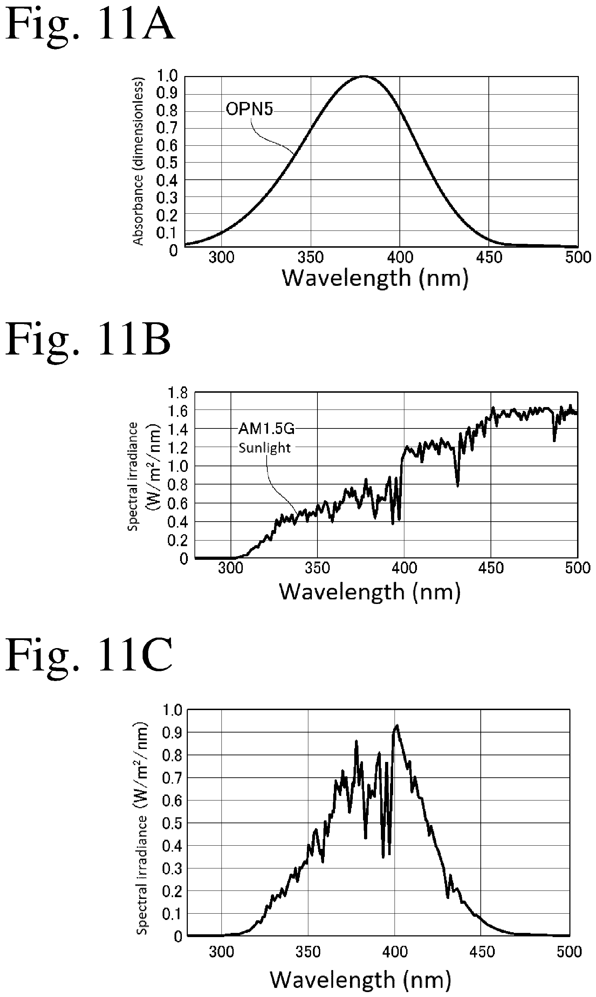

Further, in the calculation unit, as illustrated in Fig. 11, a reference effective spectral irradiance (W/m²/nm) found from a product of a spectral irradiance (W/m²/nm) of a sunlight spectrum defined by AM1.5G and the OPN5 absorption spectrum, on a per wavelength basis, may be calculated and compared with the received effective spectral irradiance (W/m²/nm).

Specifically, for example, the reference effective spectral irradiance (W/m²/nm) found from the product of the spectral irradiance (W/m²/nm) of the sunlight spectrum defined by AM1.5G and a relative absorbance of the OPN5 absorption spectrum, on a per wavelength basis, is calculated. Additionally, the spectrum of the light received by the irradiated subject can be calculated, as the received effective spectral irradiance (W/m²/nm), as the product of the spectral irradiance of each wavelength of the wavelength region of 280 nm to 500 nm of the light and a relative absorbance of data of the OPN5 absorption spectrum, on a per wavelength basis. In a comparison between the calculated reference effective spectral irradiance and the calculated received effective spectral irradiance, the received effective spectral irradiance can be regulated within a range of 0.2% or more of the reference effective spectral irradiance. Such a range can be considered a range within which the radiation conditions of the light can be regulated to radiate realistic light to the irradiated subject. It should be noted that, with a lower limit of the range being less than 0.2%, the radiation of the light is not enough, and thus sufficient effects may not be obtained. On the other hand, an upper limit of the range can be set to 4,000%.

The above-described range is based on the reference effective spectral irradiance found from the product of the spectral irradiance of the sunlight spectrum defined by AM1.5G and the OPN5 absorption spectrum, on a per wavelength basis, and is obtained by comparing the reference effective spectral irradiance with the received effective spectral irradiance calculated as the product of the spectral irradiance (spectral irradiance of each wavelength in the wavelength region of 280 nm to 500 nm) of the spectrum of the actually received light and the relative absorbance of the data of the OPN5 absorption spectrum, on a per wavelength basis, as [Received effective spectral irradiance] / [Reference effective spectral irradiance] x 100. Such a comparison allows for a generalized evaluation of the degree (percentage: %) of the actual received effective spectral irradiance relative to the common reference effective spectral irradiance, regardless of the measurement region or the measurement time. On the basis of the calculated results, it is possible to clearly define the objective relationship with the physical effects through OPN5.

Fig. 12 is a graph contrasting the reference effective spectral irradiance (reference sign b) shown in Fig. 11 (C) and the received effective spectral irradiance (reference sign a) shown in Fig. 9 (C). Thus, for example, the received effective spectral irradiance obtained in Fig. 9 can be evaluated by comparison with the reference effective spectral irradiance (W/m²/nm) found from the product of the spectral irradiance of the sunlight spectrum defined by AM1.5G at each wavelength and the OPN5 absorption spectrum, on a per wavelength basis. This allows for a generalized evaluation of the degree (percentage: %) of the actual received effective spectral irradiance relative to the common reference effective spectral irradiance, regardless of the measurement region or the measurement time. The results shown in this Fig. 12 indicate that, for example, the effective irradiance integrated within the wavelength range of 280 to 500 nm is 57 W/m² for sunlight (reference sign b) and 3.3 W/m² for violet light (reference sign a) and, in a case in which the effective irradiance of sunlight is standardized as 100%, the effective irradiance of violet light can be evaluated as 6%. Thus, by a contrast with the case of the effective irradiance of sunlight being standardized as 100%, it is possible to clearly define the objective relationship between the contrast results and the physical effects through OPN5. Further, the received effective irradiance obtained can be compared not only with the reference effective irradiance of sunlight, but also with the received effective irradiance obtained by other light source systems, and can be compared between different light sources.

It should be noted that "AM1.5G" is sunlight data that has been adopted at the standard level in solar cell development and the like, and "AM" means air mass. This AM1.5G has been standardized by the American Society for Testing and Materials (ASTM) as indicated at the URL "https://unit.aist.go.jp/rp-envene2022/PV/ja/about_pv/output/measure.html," and the data is published at "https://tinystones.net/pv/pv04.html" (the National Renewable Energy Laboratory (NREL) of the United States Department of Energy; "https://www.nrel.gov/grid/solar-resource/spectra.html").

From Fig. 11 (c), it can be seen that a maximum value of the effective spectral irradiance is 0.93 W/m²/nm (value at 402 nm). The effective irradiance, as mentioned above, has an integrated value of 57 W/m² in the wavelength region of 280 to 500 nm. An effective dose when exposed to AM1.5G sunlight for three hours per day is a value obtained by multiplying the 57 W/m² described above by 10,800 seconds, which is understood to be 615,600 J/m² (= 615.6 kJ/m²). It should be noted that, given approximately 1 W/m²/nm as the maximum value of the effective spectral irradiance, 10 W/m²/nm, which is 10 times thereof, can be set as the maximum value. The effective irradiance, assuming a time when this effective spectral irradiance is a square-type spectrum, can be calculated as 10 W/m²/nm x 220 nm = 2,200 W/m², and the effective dose in a case of radiance for three hours per day at this effective irradiance can be set as 21,600,000 J/m² (= 21.6 MJ/m²).

As described above, an effective irradiance integrated within the wavelength range of 280 to 500 nm was 57 W/m² for sunlight and 3.3 W/m² for violet light. For such values, examples of minimum values include 0.01 W/m²/nm for the effective spectral irradiance, 0.1 W/m² for the effective irradiance, and 1,000 J/m² for the effective dose. Further, examples of maximum values include 10 W/m²/nm for the effective spectral irradiance, 2,200 W/m² for the effective irradiance, and 21.6 MJ/m² (M stands for million) for the effective dose. It should be noted that the effective irradiance is a value in a case of radiance for three hours.

The light spectral data can be calculated by comparison in all or part of a specific wavelength region of the OPN5 absorption spectrum. Thereby, even in a case in which the received light includes various light spectral data, it is possible to calculate the light spectral data by comparison with all or part of the wavelength region of the OPN5 absorption spectrum, and thus apply the method to any evaluation in which the wavelength region of the light is specified using a filter or the like. As a result, the method can be utilized as a measurement device for clearly defining whether the physical effects through OPN5 are related to the received effective spectral irradiance across the entire wavelength region of the OPN5 absorption spectrum, related to the received effective spectral irradiance in a specific wavelength region of the OPN5 absorption spectrum, or the like. It should be noted that, for example, as a method of specifying a wavelength region of the light by using a filter or the like, it is also possible to specify a specific wavelength region such as that of violet light or the like having the wavelength region of 360 to 400 nm, for example.

In the example described above, the spectral irradiance of the spectrum of the light actually received is set as the spectral irradiance of each wavelength in the wavelength region of 280 nm to 500 nm, but the wavelength range may be set within a specific range. For example, in the calculation unit, during comparisons in the wavelength region, the received effective spectral irradiance may be determined by integration in the wavelength region (W/m²), and integration of the received effective irradiance and integration of the reference effective spectral irradiance in the same wavelength region may be performed. The results may then be compared and evaluated.

For the comparison of the integration results, the effective irradiance (W/m²) obtained by integrating the obtained results within a specific wavelength range can be compared between, for example, the reference effective irradiance of sunlight and the received effective irradiance of violet light, and contrasted as the received effective irradiance of violet light relative to the reference effective irradiance of sunlight.

Furthermore, in the calculation unit, it is possible to evaluate a radiation duration dependency of the light with respect to the effects on the body imparted by the absorption of light by the body through OPN5 by comparing (for example, percentage: %) a received effective dose (J/m²), obtained by multiplying the light effective irradiance (W/m²) by the radiation duration (seconds), and a received reference effective dose (J/m²), obtained by multiplying the reference effective irradiance (W/m²) by the radiation duration (seconds). It should be noted that the dose is the amount of light of exposure per day, regardless of whether the exposure duration is short (for example, in a case of being briefly exposed to strong flash of light) or the exposure time with weak light is long. On the other hand, the time of day (time frame) of exposure is not particularly limited, but in practice exposure during the daytime is more effective.

With such evaluations based on the effective irradiance and the effective dose, it is possible to quantitatively evaluate the effects on the body imparted by the absorption of light by the body through OPN5. Such a relationship is clearly defined, making it possible to, for example, evaluate what wavelength range of light, irradiance level, radiation time frame, and radiation duration are effective in relation to the physical effects of OPN5.

The measurement unit can be exemplified by, for example, a sensor 12 illustrated in Figs. 8 (A) and 8 (B), and is preferably provided to eyeglasses, an earphone, a wristwatch, a chest badge, or other article worn on the body, worn on the body itself by adhesion or the like, or attached to an installation near or around the body. Attachment in such a mode is selected and performed as desired on the basis of the installation form (light source form, light source installation position, and the like) of the light source that radiates the light, and thus is not likely to interfere with daily life and makes it easy to measure the light spectral data received by the eyes and the body.

### (Measurement of spectral data)

Spectral data of light can typically be measured using a spectrometer or an ultraviolet (UV) meter (also called a power meter). A spectrometer can measure and obtain spectral irradiance and, when the result is integrated with respect to the wavelength, the irradiance is found. Integration is performed in a wavelength region of a specific range, making it possible to express the result as the irradiance in the wavelength region, and thus preferably use such a method. On the other hand, a UV meter can directly display the irradiance. For example, a commercially available UVA meter detects light of 320 to 400 nm and displays the irradiance in the wavelength region, which is convenient. However, from a quantitative perspective, a UV meter calibrated in accordance with the used sensor is preferred. Accordingly, a UV meter cannot measure spectral information (wavelength dependency), making application to the various spectral quantities in the present invention difficult. It should be noted that the "dose" is simply determined by multiplying the irradiance by the radiation duration (seconds).

### [Light Radiation System]

The light radiation system includes the first to third modes of (1) to (3) described above.

### (First light radiation system)

A first light radiation system according to the present invention includes one or two or more light sources that radiate light having a wavelength in a wavelength region overlapping a peak wavelength of the OPN5 absorption spectrum. One or two or more conditions selected from an installation position, a radiation direction of the light, a wavelength range of the light, an irradiance of the light, an irradiance time frame of the light, and a radiation duration of the light of each of the one or two or more light sources are regulated. According to this first light radiation system, each light source is regulated in terms of any one of the above, making it possible to radiate light having a wavelength in a wavelength region overlapping the peak wavelength of the OPN5 absorption spectrum to an irradiated subject. As a result, the first light radiation system is advantageous to the activation of OPN5 present in the retina of the person who is the irradiated subject.

A second light radiation system according to the present invention includes one or two or more light sources that radiate light having a wavelength in a wavelength region overlapping a peak wavelength of an OPN5 absorption spectrum. A spectrum of the light received by an irradiated subject is regulated so that a received effective spectral irradiance (W/m²/nm) calculated as a product of a spectral irradiance (W/m²/nm) of each wavelength in a wavelength region of 280 nm to 500 nm of the light and a relative absorbance of data of the OPN5 absorption spectrum, on a per wavelength basis, is 0.2% or more of a reference effective spectral irradiance (W/m²/nm) calculated as a product of a spectral irradiance (W/m²/nm) of a sunlight spectrum defined by AM1.5G and a relative absorbance of the OPN5 absorption spectrum, on a per wavelength basis. According to this second light radiation system, the spectrum of the light received by the irradiated subject is regulated so that the received effective spectral irradiance is 0.2% or more of the reference effective spectral irradiance. As a result, the second light radiation system is advantageous to the activation of OPN5 present in the retina of the person who is the irradiated subject. The light having the irradiance that is a percentage within the above-described range is calculated on the basis of the light spectrum measured at the irradiated subject or a position around the irradiated subject.

### (Third light radiation system)

A third light radiation system according to the present invention includes a light radiating means including one or two or more light sources that radiate light having a wavelength in a wavelength region overlapping a peak wavelength of an OPN5 absorption spectrum, a light measuring means for measuring spectral data of the light and comparing the spectral data of the light and data of the OPN5 absorption spectrum. One or two or more conditions selected from an installation position, a radiation direction of the light, a wavelength range of the light, an irradiance of the light, a radiation time frame of the light, and a radiation duration of the light of each of the one or two or more light sources are regulated on the basis of results obtained by the comparison. According to this third light radiation system, the installation position, the radiation direction of the light, the wavelength range of the light, the irradiance, the radiation time frame, and the radiation duration of each light source are regulated on the basis of the results obtained through comparison in the light measurement method. As a result, the third light radiation system is advantageous to the activation of OPN5 present in the retina of the person who is the irradiated subject.

The components of these first to third light radiation systems have already been described, and thus description thereof is omitted below, and elements that have not been described are described below.

### (Light sensor)

In the light radiation system, the received effective spectral irradiance actually received by the eyes is specified, and thus the substantial spectral irradiance of the light received by the eyes is measured through measurement with a light sensor. Examples of the light sensor include GaAsP photodiodes, and these can be selected and applied as desired. Such a light sensor 12 may be, for example, the light sensor 12 installed on eyeglasses as illustrated in Fig. 8, the light sensor 12 in the mode of a chest badge, or a light sensor installed around the eyes, and is not particularly limited. A means for providing the light sensor on the eyeglasses, a means for providing the light sensor around the eyes, and the like are not particularly limited. The light sensor may be directly fixed to the eyeglasses or hooked using a hook or the like. Further, the light sensor may be fixed to an ear hooking accessory or the like and hooked to the ear. As described in the section describing the measurement of the spectral data, this light sensor preferably includes a spectrometer.

### (Installation form of light source and regulation of light)

In the light radiation system, the installation form of the light source (including an arrangement form in a case in which a diffusing plate or a reflecting plate is provided as necessary) and the regulation of the light delivered to the eyes are important. Through the installation form of the light source and the regulation of the light described below, it is possible to regulate the light that reaches the eyes. Modes of the regulation of the light include a regulating means related to the radiation conditions of the light and a regulating means for the directionality of the light.

For the "regulating means related to the radiation conditions of the light," for example, the light emitted from the one or two or more light sources is preferably regulated by one or two or more means selected from setting or changing the wavelength range, setting or changing the irradiance, setting or changing the time frame, and setting or changing the radiation duration.

Regarding "setting or changing the wavelength range," "setting a specific wavelength range" refers to setting the wavelength range of the light radiated from the light source. Examples include a means such as providing a light source that emits light having a wavelength within a specific wavelength range, or providing a filter that transmits a specific wavelength range in front of the light source to emit light having a wavelength within the specific wavelength range. Further, "changing a specific wavelength range" refers to changing the wavelength range of the light radiated from the light source. Examples include a means such as separately providing light sources that emit light having wavelengths within different wavelength ranges and changing the light source by switching between the light sources, or switching to another filter, in front of the light source, that transmits a specific wavelength range to transmit light having a wavelength within a different wavelength range.

"Setting or changing the irradiance" refers to setting or changing the irradiance of the light emitted from the light source to a desired value. Such setting or changing of the irradiance is preferably performed in accordance with the level of the received effective spectral irradiance received by the eyes. In a case in which sufficient received effective spectral irradiance is received from the perspective of the light effect of OPN5 evaluated on the basis of the light measuring means of the present invention, the radiation may be stopped or the irradiance may be decreased. On the other hand, in a case in which insufficient received effective spectral irradiance is received from the perspective of the light effect of OPN5 evaluated on the basis of the light measuring means of the present invention, the irradiance may be increased. Specifically, for example, in a case in which a violet light source that emits violet light having a wavelength of 360 to 400 nm is used, the lower limit of the irradiance can be set to preferably 0.05 W/m² (5 µW/cm²), more preferably 0.10 W/m², and even more preferably 0.2 W/m². The upper limit of the irradiance can be set to preferably 2,000 W/m², more preferably 20 W/m², and even more preferably 10 W/m². The actual range of the irradiance may be within a range combining the lower limit and the upper limit described above, and the irradiance is preferably set and changed as desired within such a range.

"Setting or changing the time frame" refers to setting or changing the time frame of emitting the light from the light source as desired. The setting or the changing of the time frame is preferably performed in accordance with the time frame in which the light is to be radiated from the perspective of the light effect of OPN5 evaluated on the basis of the light measuring means of the present invention. Specifically, for example, in a case of the evening following sunset, the light within the range of the OPN5 absorption spectrum cannot be obtained from the sun outdoors and thus, in the time frame of the evening following sunset, a setting or a change of radiating light indoors is desirably not performed from the perspective of not disrupting a circadian rhythm. Further, the time frame can be set as desired, and thus may be a regular or an intermittent time frame.

"Setting or changing the radiation duration" refers to setting or changing the radiation duration of emitting the light from the light source as desired. The setting or the changing of the radiation duration is preferably performed in accordance with a length of the radiation duration of the light from the comparison results based on the light measuring means of the present invention. Specifically, for example, the light may be radiated intermittently or discontinuously, for example. It should be noted that, in the case of setting or changing the radiation duration, the irradiance is preferably also set in conjunction thereto.

Regarding the "regulating means for the directionality of the light," to ensure that the light emitted from the one or two or more light sources is directed toward the eyes, the light emitted from each light source is preferably regulated by one or two or more means selected from installing or changing the light source (including installing or changing the light source in a case in which a diffusing plate or a reflecting plate is provided as necessary); setting or changing the angle of the light emitted from the light source; in a case in which a diffusing material that diffuses the light is provided, installing or changing the diffusion angle of the diffusing material or the light source utilizing the diffusing material; and, in a case in which a reflecting material that reflects the light is provided, regulating the reflection angle of the reflecting material and installing or changing the light source utilizing the reflecting material, for example. This mode is a regulating means for the directionality of the light. "Directionality" is an important factor from the perspective of the light effects of OPN5. For example, in the case of deskwork, the light source is particularly desirably positioned to radiate in the direction of the eyes ahead in the visual line. Further, as illustrated in Fig. 7, preferably the orientation of the light can be changed automatically or manually from "D" to "D'."

Regarding "installing or changing the light source," "setting or changing the angle of the light emitted from the light source," "installing or changing the diffusing material that diffuses the light," and "installing or changing the reflecting material that reflects the light," in all cases, preferably each light source is installed in advance so that the orientation of the light is in the direction of the eyes and installed so that the orientation can be changed to a desired direction, as mentioned above.

### (Application)

The first to third light radiation systems according to the present invention are advantageous to the activation of OPN5 present in the retina of the person who is the irradiated subject. The implementation of such light radiation systems improves visual functions and non-visual functions, suppresses the progression of myopia (for example, suppresses elongation of an axial length of the eye), suppresses an onset of myopia, and improves a thickness of the choroid, effects on cerebral blood flow and the nervous system, and effects on others (testes, skin, and the like), and can be expected to contribute to the pursuit and development of research in these areas.

### Descriptions of Reference Numerals

- 10: Light source
- 11: Reflecting material
- 12: Sensor
- D, D': Orientation of light
- 21: Light measuring means
- 22: Measurement unit
- 23: Calculation unit
- 24: Memory
- 25: Output unit (Display unit)

## Claims

1. A light radiation system comprising:
one or two or more light sources that radiate light having a wavelength in a wavelength region overlapping a peak wavelength of an OPN5 absorption spectrum,
one or two or more conditions selected from an installation position, a radiation direction of the light, a wavelength range of the light, an irradiance of the light, a radiation time frame of the light, and a radiation duration of the light of each of the one or two or more light sources being regulated.

2. A light radiation system comprising:
one or two or more light sources that radiate light having a wavelength in a wavelength region overlapping a peak wavelength of an OPN5 absorption spectrum,
a spectrum of the light received by an irradiated subject being regulated so that a received effective spectral irradiance (W/m²/nm) calculated as a product of a spectral irradiance (W/m²/nm) of each wavelength in a wavelength region of 280 nm to 500 nm of the light and a relative absorbance of data of the OPN5 absorption spectrum, on a per wavelength basis, is 0.2% or more of a reference effective spectral irradiance (W/m²/nm) calculated as a product of a spectral irradiance (W/m²/nm) of a sunlight spectrum defined by AM1.5G and a relative absorbance of the OPN5 absorption spectrum, on a per wavelength basis.

3. A light radiation system comprising:
a light radiating means including one or two or more light sources that radiate light having a wavelength in a wavelength region overlapping a peak wavelength of an OPN5 absorption spectrum; and
a light measuring means for measuring spectral data of the light and comparing the spectral data of the light and data of the OPN5 absorption spectrum,
one or two or more conditions selected from an installation position, a radiation direction of the light, a wavelength range of the light, an irradiance of the light, a radiation time frame of the light, and a radiation duration of the light of each of the one or two or more light sources being regulated on the basis of results obtained by the comparison.

4. The light radiation system according to any one of claims 1 to 3, wherein
the light is light including 380 nm that is the peak wavelength of the OPN5 absorption spectrum.

5. The light radiation system according to claim 4, wherein
the light has a wavelength of 400 nm or less or 410 nm or less.

6. The light radiation system according to any one of claims 1 to 3, wherein
the one or two or more light sources are disposed at one or two or more positions selected from a position where the light is radiated toward an irradiated subject who is a radiation target, a position in a field of view of the irradiated subject, a position along the subject' s visual line, a position where the light is radiated to the irradiated subject as diffused light diffused by a diffusing material, and a position where the light is radiated to the irradiated subject as reflected light reflected by a reflecting material.

7. The light radiation system according to claim 6, wherein,
for each of the one or two or more light sources,
(i) in a case in which the light source is provided at a position where the light is radiated toward the irradiated subject, the irradiance of the light is regulated in accordance with a distance and/or an angle from the light source to the irradiated subject,
(ii) in a case in which the light source is provided at a position in the field of view of the irradiated subject or a position in the visual line direction from the irradiated subject side, the irradiance of the light is regulated in accordance with the distance and/or the angle from the light source to the irradiated subject,
(iii) in a case in which the light source is provided at a position where the light is radiated to the irradiated subject as the diffused light diffused by the diffusing material, the irradiance of the light is regulated in accordance with a distance and/or an angle from the light source and the diffusing material to the irradiated subject, or a diffusion angle and/or a transmittance of the diffusing material, and
(iv) in a case in which the light source is provided at a position where the light is radiated to the irradiated subject as the reflected light reflected by the reflecting material, the irradiance of the light is regulated in accordance with a distance and/or an angle from the light source and the reflecting material to the irradiated subject, or a reflection angle and/or a reflectance of the reflecting material.

8. The light radiation system according to any one of claims 1 to 3, wherein
each of the one or two or more light sources is selected from a structure-attached light source attached to a floor (including underfoot), a wall, a ceiling (including a suspended ceiling), or other structure; an installation-attached light source attached to a desk, a table, a partition (divider, screen), a shelf, a desktop member, a personal computer (PC), a display, a PC keyboard, a television, an audio device, or other equipment or installation; a built-in or attached light source of a portable PC, a tablet, a smartphone, or other mobile terminal; a body-accessory-attached light source detachably mounted or attached to a vision correcting aid, eye protective equipment, face protective equipment, neck hanging accessory, ear hooking accessory, head-attached equipment, or other body accessory; and a functional device-attached light source attached to a functional device for augmented reality (AR), virtual reality (VR), or mixed reality (MR) (including goggles, a headset, and other functional devices).

9. The light radiation system according to any one of claims 1 to 3, wherein
the one or two or more light sources are regulated in orientation or position manually or automatically.

10. The light radiation system according to claim 3, wherein
a received effective spectral irradiance (W/m²/nm) found from a product of the spectral data (W/m²/nm) of the light and the data of the OPN5 absorption spectrum, on a per wavelength basis, is calculated.

11. The light radiation system according to claim 10, wherein
a reference effective spectral irradiance (W/m²/nm) found from a product of a spectral irradiance (W/m²/nm) of a sunlight spectrum defined by AM1.5G and the OPN5 absorption spectrum, on a per wavelength basis, is calculated and compared with the received effective spectral irradiance (W/m²/nm).

12. The light radiation system according to claim 3, wherein
the spectral data of the light is calculated by comparison in all or part of a wavelength region of the OPN5 absorption spectrum.

13. The light radiation system according to claim 12, wherein,
during comparison in the wavelength region, the received effective spectral irradiance (W/m²/nm) is integrated in the wavelength region to obtain a received effective irradiance (W/m²), the reference effective spectral irradiance (W/m²/nm) is similarly integrated in the same wavelength region to obtain a reference effective irradiance (W/m²), and the received effective irradiance and the reference effective irradiance thus obtained are compared.

14. The light radiation system according to claim 13, wherein
a received effective dose (J/m²) obtained by multiplying the received effective irradiance (W/m²) by the radiation duration (seconds) and a received reference effective dose (J/m²) obtained by multiplying the reference effective irradiance (W/m²) by the radiation duration (seconds) are compared.

15. The light radiation system according to claim 3, wherein
the measurement is performed by a measurement unit, and the measurement unit is provided to eyeglasses, an earphone, a wristwatch, a chest badge, or other article worn on a body, worn on the body itself, or attached to an installation near or around the body, on the basis of a form of each of the one or two or more light sources that radiate the light.

16. A light radiation method comprising:
radiating, to an irradiated subject, light having a wavelength in a wavelength region overlapping a peak wavelength of an OPN5 absorption spectrum from one or two or more light sources that radiate the light;
comparing spectral data of the light obtained by receiving the radiated light and data of the OPN5 absorption spectrum; and
regulating radiation conditions of the light (one or two or more conditions selected from an installation position, a radiation direction of the light, a wavelength range of the light, an irradiance of the light, a radiation time frame of the light, and a radiation duration of the light of each of the one or two or more light sources) on the basis of results of the comparison.

17. The light radiation method according to claim 16, further comprising:
a light measuring means for measuring the spectral data of the light and comparing the spectral data of the light and the data of the OPN5 absorption spectrum,
a received effective spectral irradiance (W/m²/nm) found from a product of the spectral data of the light and the data of the OPN5 absorption spectrum, on a per wavelength basis, being calculated.

18. A light measurement method comprising:
measuring spectral data of light having a wavelength in a wavelength region overlapping a peak wavelength of an OPN5 absorption spectrum;
comparing the measured spectral data of the light and data of the OPN5 absorption spectrum stored in advance; and
displaying results of the comparison on a display device or outputting the results as data.

19. The light measurement method according to claim 18, wherein
the comparison is performed by a calculation unit, and a received effective spectral irradiance (W/m²/nm) found from a product of the spectral data of the light and the data of the OPN5 absorption spectrum, on a per wavelength basis, is calculated in the calculation unit.

20. The light measurement method according to claim 19, wherein
a reference effective spectral irradiance (W/m²/nm) found from a product of a spectral irradiance (W/m²/nm) of a sunlight spectrum defined by AM1.5G and the OPN5 absorption spectrum, on a per wavelength basis, is calculated and compared with the received effective spectral irradiance (W/m²/nm).

21. The light measurement method according to claim 18 or 19, wherein
the spectral data of the light is compared in all or part of a wavelength region of the OPN5 absorption spectrum.

22. The light measurement method according to claim 21, wherein,
during comparison in the wavelength region, the received effective spectral irradiance is integrated in the wavelength region to obtain a received effective irradiance (W/m²), the reference effective spectral irradiance is similarly integrated in the same wavelength region to obtain a reference effective irradiance (W/m²), and the received effective irradiance and the reference effective irradiance thus obtained are compared.

23. The light measurement method according to claim 22, wherein
a received effective dose (J/m²) obtained by multiplying the received effective irradiance (W/m²) by a radiation duration (seconds) and a received reference effective dose (J/m²) obtained by multiplying the reference effective irradiance (W/m²) by the radiation duration (seconds) are compared.

24. The light measurement method according to claim 18 or 19, wherein
the measurement is performed by a measurement unit, and the measurement unit is provided to eyeglasses, an earphone, a wristwatch, a chest badge, or other article worn on a body, worn on the body itself, or attached to an installation near or around the body, on the basis of a form of each of the one or two or more light sources that radiate the light.

25. A light measurement device comprising:
a measurement unit that measures spectral data of light having a wavelength in a wavelength region overlapping a peak wavelength of an OPN5 absorption spectrum;
compares the spectral data of the light measured by the measurement unit and data of the OPN5 absorption spectrum stored in advance; and
displays results of the comparison on a display device or outputs the results as data.
